(19) **Europäisches Patentamt**
**European Patent Office**
**Office européen des brevets**

(11) **EP 4 429 553 B1**

(12) **EUROPEAN PATENT SPECIFICATION**

(45) Date of publication and mention
of the grant of the patent:
**24.12.2025  Bulletin 2025/52**

(21) Application number: **22813122.3**

(22) Date of filing: **27.10.2022**

(51) International Patent Classification (IPC):
**A61B 6/00** *(2024.01)*    **G16H 40/40** *(2018.01)*
**H05G 1/54** *(2006.01)*

(52) Cooperative Patent Classification (CPC):
**A61B 6/40; A61B 6/586; G16H 40/40; H05G 1/54**

(86) International application number:
**PCT/EP2022/080093**

(87) International publication number:
**WO 2023/078777 (11.05.2023 Gazette 2023/19)**

(54) **USAGE-BASED WEAR MODEL AND IMPROVED LIFETIME FOR X-RAY DEVICES**

NUTZUNGSBASIERTES VERSCHLEISSMODELL UND VERBESSERTE LEBENSDAUER FÜR RÖNTGENVORRICHTUNGEN

MODÈLE D'USURE BASÉ SUR L'UTILISATION ET DURÉE DE VIE AMÉLIORÉE POUR DISPOSITIFS À RAYONS X

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority:  **08.11.2021   US 202163276688 P**

(43) Date of publication of application:
**18.09.2024   Bulletin 2024/38**

(73) Proprietor: **Koninklijke Philips N.V.**
**5656 AG Eindhoven (NL)**

(72) Inventors:
• **KORST, Johannes, Henricus, Maria**
**5656 AG Eindhoven (NL)**
• **SOSSIN, Artur**
**5656 AG Eindhoven (NL)**

• **GRAESSLIN, Ingmar**
**5656 AG Eindhoven (NL)**
• **PRONK, Serverius, Petrus, Paulus**
**5656 AG Eindhoven (NL)**
• **DEMEWEZ, Tiblets, Zeray**
**5656 AG Eindhoven (NL)**
• **SPRONG, Hans, Peter**
**5656 AG Eindhoven (NL)**
• **BARBIERI, Mauro**
**5656 AG Eindhoven (NL)**

(74) Representative: **Philips Intellectual Property & Standards**
**High Tech Campus 52**
**5656 AG Eindhoven (NL)**

(56) References cited:
**WO-A1-2020/193103      US-A1- 2019 317 144**
**US-B1- 6 212 256**

## Description

### FIELD

The following relates generally to the medical imaging arts, X-ray imaging arts, X-ray tube arts, X-ray tube maintenance arts, and related arts.

### BACKGROUND

The X-ray tube is an expensive component of any medical X-ray imaging machine, and X-ray tubes have limited useful lifetimes of anywhere from a couple years to a decade or longer depending on installation-specific factors such as how intensively the X-ray imaging machine is used. Each X-ray tube also has individual performance characteristics. Hence, X-ray tube monitoring and maintenance is commonly performed.

A common aspect of X-ray tube monitoring and maintenance is to provide automated estimation of the remaining useful life (RUL), or equivalently a time-to-failure prediction or estimated time remaining until end-of-life (EOL), for the X-ray tube. This estimate can be based simply on X-ray tube usage hours. 'One of the most common root causes of X-ray tube failure is the wear out of the tube's filament. However, filament wear depends on more than just the tube usage hours - for example, running the X-ray tube at a higher filament current will accelerate the filament wear. Hence, in the monitoring of some X-ray imaging machines, a more advanced predictive model is employed, such as artificial intelligence (AI) modeling of time remaining until EOL based on historical X-ray tube performance information. Modern X-ray imaging machines are computerized and often maintain a machine log of key operational parameters and operating history, usually including information on X-ray tube voltage, filament current, and tube current that have been applied in the successive runs of the X-ray tube, among other data. The machine log can thus be mined for data to be input to the AI modeling to generate the estimated time remaining until EOL. In a common scenario, the estimated time remaining until EOL is displayed on a workstation of a remote monitoring engineer and/or on the X-ray imaging machine control console when the estimated time remaining until EOL falls below some threshold (e.g., on the order of one or two weeks). Such a prediction beneficially allows the X-ray imaging machine operator to plan ahead for replacing the X-ray tube, ideally during a time interval of low usage or during some other scheduled machine downtime.

Accuracy of the time remaining until EOL estimation is beneficial. If the estimated time remaining until EOL is erroneously long, then the X-ray tube may fail before it has been replaced. In this case, the X-ray imaging machine may become unusable until the failed X-ray tube is replaced. This can have serious adverse consequences, most notably including delayed or canceled patient imaging sessions and consequent financial losses for the medical institution. X-ray tube failure can also produce electrical stress and/or damage to ancillary components of the X-ray imaging machine, making the maintenance more complex and costly. On the other hand, an estimated time remaining until EOL that is erroneously short may lead to the X-ray tube being replaced too early, which can lead to additional cost. In some examples, an X-ray tube for medical applications typically has two filaments albeit with different technical specifications, where depending on the type of examination, one of the two would be the preferred filament in terms of duration or image quality. If one filament fails, then the other could still be used, but that would not be optimal for some examinations. Furthermore, during the time that the broken filament is not yet replaced, the second filament may also fail during a medical intervention, which may imply that a patient is at risk. Hence, it is recommended that a broken filament is replaced as quickly as possible, while in the meantime the X-ray tube is used as little as possible.

The following discloses certain improvements to overcome these problems and others.

A document US 2019/317144 A1 relates to generating one or both of a failure prediction indication for an X-ray tube or a remaining useful life estimate for the X-ray tube. In one implementation, a trained static tube model is used in estimating health (e.g., thickness) of the electron emitter of the X-ray tube, which in turn may be used in predicting remaining useful life of an electron emitter of the X-ray tube.

A document WO 2020/193103 A1 discloses a method of setting a filament demand in an x- ray apparatus. The x-ray apparatus has a filament, through which the passing of a heating current allows thermionic emission of electrons from the filament. The x-ray apparatus has a target, arranged to generate x-rays from the electrons emitted from the filament. The x-ray apparatus has a detector, arranged to detect x-rays generated by the target for forming an x-ray image. The x-ray apparatus has a controller configured to perform a measurement operation of the x-ray apparatus. The measurement measures a parameter of the x-ray apparatus. The controller is configured to set a filament demand for the filament. The filament demand correlates with the current passed through the filament. The method comprises varying the filament demand between a first value corresponding to a lower filament current and a second value corresponding to a higher filament current. The method comprises measuring the parameter at a series of values of the filament demand between the first value and the second value. The method comprises detecting a knee in the measured parameter. The method comprises determining the filament demand corresponding to the detected knee in the parameter. The method comprises setting the filament demand for the x-ray apparatus based on the determined filament demand corresponding to the detected knee in the parameter.

## SUMMARY

In some embodiments disclosed herein, a non-transitory computer readable medium stores instructions executable by at least one electronic processor to perform a method of monitoring a component of a medical device. The medical device is a medical imaging device, and the component is an X-ray tube of the medical imaging device. The method includes retrieving information about a filament current correction factor applied during operation of the component from the medical device, the filament current correction factor being a multiplication factor for adjusting a filament current of the X-ray tube in order to compensate for a varying filament resistance so as to retain a desired X-ray tube current; deriving a wear metric indicative of wear of a portion of the component from the retrieved information about the correction factor applied during the operation of the component, the wear metric comprising a time derivative of the filament current correction factor; estimating a time remaining until end-of-life (EOL) of the component based on the wear metric; and outputting an alert indicating estimated time remaining until EOL of the component.

The method may further compriseretrieving information about a medical procedure to be performed with the medical device and using the component; selecting values for one or more operating parameters of the component of the medical device based on the estimate of the time remaining until EOL of the component and the retrieved information about the current medical procedure; and adjusting the one or more parameters of the component of the medical device to the selected values.

One advantage resides in performing a manual calibration to optimize a lifetime of an X-ray tube only when appropriate.

Another advantage resides in providing a more accurate RUL estimate for an X-ray tube.

Another advantage resides in predicting a failure time of an X-ray tube to determine an optimal servicing time for an X-ray device, thereby reducing a down time of the X-ray device.

Another advantage resides in reducing delayed or cancelled X-ray examinations.

Another advantage resides in decreased financial losses for a medical institution based on accurately determining a predicted failure date of an X-ray tube.

A given embodiment may provide none, one, two, more, or all of the foregoing advantages, and/or may provide other advantages as will become apparent to one of ordinary skill in the art upon reading and understanding the present disclosure.

## BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

FIGURE 1 diagrammatically illustrates an X-ray imaging device in accordance with the present disclosure.

FIGURE 2 diagrammatically illustrates an X-ray device monitoring method using the X-ray imaging device of FIGURE 1.

## DETAILED DESCRIPTION

The following discloses leveraging a filament current adjustment factor, such as a c-factor used in current X-ray systems, to estimate the time remaining until end-of-life (EOL) of the X-ray tube.

In an X-ray tube, a filament (i.e., a cathode), typically made of tungsten or another refractory metal, is heated to such high temperatures that electrons escape from the filament into the surrounding vacuum. This process is called thermionic emission. By applying a high voltage between the cathode and the anode, the so-called tube voltage, the electrons that have escaped from the cathode are accelerated towards the anode as a collimated electron beam, forming the so-called tube current. When the electron beam strikes the anode, X-rays are generated. The temperatures for thermionic emission are high enough that tungsten atoms evaporate into the surrounding vacuum, which results in decreasing the radius of the filament wire over time.

The filament of an X-ray tube does not wear evenly. Over time, so-called hot spots are created where the electron emission as well as the tungsten evaporation is considerably higher than on other locations of the filament. Hot spots may develop due to differences in the polycrystalline structure of the filament and due to other local differences. At a hot spot, the local temperature is higher, which results in a locally higher tungsten evaporation, which, in turn, results in a local higher increase of the resistance, which results in a higher temperature, thus creating positive feedback that continues to accelerate the evaporation of the tungsten filament at the hot spot over the course of time. Hence, the wear rate will be higher near the end of life when hot spots have developed more extensively.

To account for filament wear, the medical imaging device is configured to operate the X-ray tube, including adjusting a filament current of a filament of the X-ray tube based on a correction factor, or c-factor. The filament correction factor is a multiplication factor that is used to adjust the filament current to compensate for the varying filament resistance, to retain a desired tube current. The c-factor (c) can be updated by a dedicated calibration, such that an appropriate start value for the filament current can be chosen for a desired tube current and tube voltage combination. Alternatively, it can be updated after each run.

It is recognized herein that the correction factor, or c-factor, used by the medical imaging device in adjusting a filament current is a useful datum for estimating the time remaining until EOL of the X-ray tube.

As further disclosed herein, the estimated time remaining until EOL of the X-ray tube can be advantageously used to adjust operation of the medical imaging device in ways that extend the X-ray tube life. If the tube quality degrades or fails, the system might deliver sub-optimal imaging results (e.g., deteriorated image quality) or may become completely unusable. The former case may lead to misdiagnosis or radiologists' productivity losses due to, e.g., more difficult image interpretation, while the latter case may result in reduced patient throughput. This may lead to financial losses to the medical institution employing the device as well as the tube manufacturer depending on the service contract provided for the X-ray tube by the tube manufacturer or system supplier. Different types of contracts are in use, ranging from full cost absorption by the customer in the case of X-ray tube failures outside of a warranty period, to renting of the X-ray tube by the customer, to lifetime service contracts in which the tube manufacturers or system suppliers offer replacement in case the usage of the tube remains within certain predefined usage margins (e.g., number of exposures per year). Certain X-ray tube operation parameters, such as tube current, exposure time or their product, kV, focal spot, and collimation within a certain boundary conditions of the available protocols, have significant impact on the tube's lifetime and greatly impact the wear of the tubes if chosen sub-optimally and in turn the tube needs to be replaced sooner. A data driven approach is disclosed herein, that allows for tube usage and life-time optimization based on, e.g., the various contract types by choosing the optimal tube control parameters. Furthermore, it enables to reduce the probability of unexpected system down-times impacting system availability as well as reduced patient throughput, which would in turn lead to a negative financial impact.

When a new X-ray tube is installed, the filament has a certain resistance. The X-ray tube is calibrated by generated a look-up table to record a measured relationship between a tube voltage and an electric current flowing through the filament (i.e., "filament current") that is effective to produce a desired amount of filament heating and consequent electron emission current to produce a reference X-ray beam intensity and a resulting tube or emission current. As the X-ray tube ages, the filament thins due to evaporation of metal from the filament. This thinning increases the filament resistance so that a lower filament current is needed to achieve the same heating and consequent reference X-ray beam intensity. A filament current adjustment factor (also referred to herein as a correction factor, or c-factor, for brevity) is applied to account for reduced filament current. In some approaches, the c-factor is multiplied against the reference electric current. As the requisite current generally decreases over time, especially as the tube continues to age, the c-factor is usually less than 1.

The c-factor starts to decrease more rapidly as the X-ray tube approaches its EOL. As previously discussed, this is believed to be due to the filament wearing non-uniformly, so that thinner regions of the filament form hot spots during use. The higher rate of change of the c-factor is disclosed herein to be used as a metric for estimating time to EOL. Various time-to-EOL models are presented that are based on the time derivative of the c-factor, ($dc/dt$), or are based on the derivative of the c-factor with respect to the filament wear, ($dc/dw$). Various models for modeling of the wear w are also disclosed. These models may be empirically generated by being parameterized and optimized with respect to training data labeled with observed (possibly accelerated) EOL dates.

These time-to-EOL estimation approaches may be degraded if they are based on wear data versus time data that has some missing data points, or if the X-ray tube goes unused for some extended time. Approaches are disclosed for detecting these situations.

The following also relates to approaches for extending the X-ray tube lifetime. These approaches are motivated by concerns relating to lifetime service contracts offered by medical device vendors. As vendors incur a cost whenever an X-ray tube is replaced under a lifetime service contract, it would be beneficial to provide for ways to extend the X-ray tube lifetime. This also reduces machine downtime and is more environmentally friendly.

The approaches for extending tube life disclosed herein are based on receiving operating information such as patient information, examination information, and so forth, and using this information to optimize the X-ray tube operating parameters (e.g., tube voltage, filament current, exposure time, whether large or small focal spot is used) to extend the X-ray tube lifetime without significantly degrading imaging examination quality. For example, if a portion of the patient moves during imaging (e.g., motion from a heartbeat of the patient), then this movement can be suppressed.

In the contemplated embodiment, the output of the filament lifetime extension analysis is implemented by automatically adjusting the configuration table of the X-ray imaging device. For example, tube parameters such as tube current, exposure time or their product, kV, focal spot, and collimation within a certain boundary conditions of the available protocols in the configuration file can be adjusted when appropriate to enforce a lower filament current and extend X-ray tube lifetime.

While primarily motivated by lifetime service contract costs, the concept could be extended to X-ray imaging devices that are serviced under contracts in which the customer bears the tube replacement cost. In this case, the customer may be informed that the X-ray tube EOL is approaching, and the disclosed approaches for extending tube life can be applied automatically (as in the previous embodiment) or provided as suggestions.

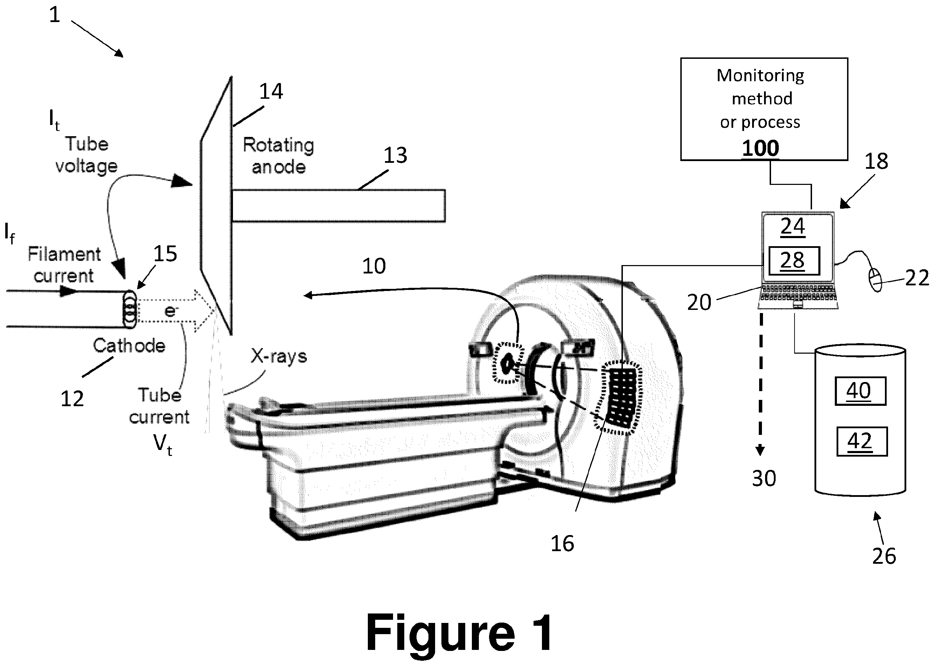

With reference to FIGURE 1, an illustrative medical device **1** is shown. The medical device **1** can be, for example, a computed tomography (CT) imaging device (as shown), or a C-arm imaging device such as are sometimes used for cardiac imaging, or an image guided therapy (IGT) system employing X-ray imaging, a fluoroscopic imaging device, a digital radiography (DR) imaging device, or other an imaging device that utilizes X-ray imaging (hereinafter referred to as an "X-ray device" or variants thereof). Even more generally, the medical device **1** can be any medical device having a component for which a manual calibration can be performed to optimize the remaining useful life (RUL) of the component, such as a linear accelerator (LINAC). As shown in FIGURE 1, the X-ray device **1** can include a component, such as an X-ray tube **10** shown by partial removal of the housing of the CT scanner **1** in FIGURE 1, and diagrammatically shown in the upper left of FIGURE 1 as having a cathode **12** and an anode **14**. The cathode **12** includes a filament **15** that when heated by a filament current $I_f$ emits electrons which can be attracted to the anode **14** by a tube voltage $V_t$ that is generated between the cathode **12** and the anode **14,** and the anode **14** (in some examples, a rotating anode that rotates about a shaft **13** to distribute heat) comprises a target area on which the electrons $e^-$ impinge, thereby generating X-rays that form an X-ray beam used during image acquisition. The flow of electrons $e^-$ forms a tube current $It$ of the X-ray tube **10**. The intensity of the X-ray beam produced by the X-ray tube **10** depends, often in a highly nonlinear fashion, on various operational parameters such as the mentioned tube voltage $V_t$, tube current, and filament current $I_f$. The diagrammatically shown X-ray tube **10** is a simplified representation - modern commercial X-ray tubes used in X-ray imaging devices often include additional components such as a grid whose geometry and electrical bias can be used to control the shape, focus, intensity, or other characteristics of the X-ray beam, and such components may introduce additional X-ray tube performance variables such as the grid voltage.

An X-ray detector **16** is configured to detect the X-ray radiation. As shown in FIGURE 1, the detector **16** typically comprises a detector array. The detector **16** is also in electronic communication with an electronic processing device **18,** such as a workstation computer, or more generally a computer. Images produced by the X-ray device **1** via the X-ray radiation generated by the cathode **12** and the anode **14** are processed by the electronic processing device **18.** The illustrative CT scanner **1** employs tomographic imaging in which the X-ray tube **10** and detector **16** rotate together around an imaging subject to acquire a three-dimensional (3D) image of the subject. In other types of X-ray imaging devices these components may be fixed in position rather than revolving around the imaging subject, and hence provide a two-dimensional (2D) image. In a C-arm configuration, the X-ray tube **10** and the detector **16** can be moved to different vantage points (called "views") around the patient to (for example) provide clinically significant views of the heart, or in an IGT to provide a chosen view of an interventional procedure. In some embodiments, the electronic processing device **18** can also serve as a device controller of the X-ray device **1.** In other embodiments, the electronic processing device **18** can also serve as a remote monitoring workstation of the X-ray device **1.**

The electronic processing device **18** may also include a server computer or a plurality of server computers, e.g., interconnected to form a server cluster, cloud computing resource, or so forth, to perform more complex computational tasks. For example, in a common configuration a local electronic processing device serves as a controller for controlling the imaging device **1** to perform image acquisition, and also to record machine log data; while a server is connected via a hospital network and/or the Internet to occasionally receive updates of the machine log data. The server performs analyses on the uploaded machine log data such as applying a predictive failure model to predict when the X-ray tube **10** will fail. Additionally, in embodiments disclosed herein, the server performs predictive analysis to determine when the operator should be alerted to cease performing manual calibration of the X-ray tube **10.** The workstation **18** includes typical components, such as an electronic processor **20** (e.g., a microprocessor), at least one user input device (e.g., a mouse, a keyboard, a trackball, and/or the like) **22,** and a display device **24** (e.g., an LCD display, plasma display, cathode ray tube display, and/or so forth). In some embodiments, the display device **24** can be a separate component from the workstation **18,** or may include two or more display devices.

The electronic processor **20** is operatively connected with one or more non-transitory storage media **26**. The non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation **18,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20**. The instructions include instructions to generate a visualization of a graphical user interface (GUI) **28** for display on the display device **24.**

The apparatus **10** is configured as described above to perform a method or process 100 of monitoring a component of a medical device. Although described herein as the medical device being the X-ray device **1** and the component being the X-ray tube **10,** the method **100** can apply for any suitable component of any suitable medical device for which a deviation from a performance of the component compared to an expected performance of the component (e.g., calibration data) can reduce the remaining useful life (RUL) of the component. As another example, a LINAC typically includes a beam-generating component in which high voltages are used to generate a beam of accelerated subatomic particles or ions, and calibrating the beam-generating component may reduce the RUL of the beam-generating component. The non-transitory storage medium **26** stores instructions which are readable and executable by the at least one electronic processor **20** to perform disclosed operations including performing the monitoring method or process **100.** In some examples, the method **100** may be performed at least in part by cloud processing.

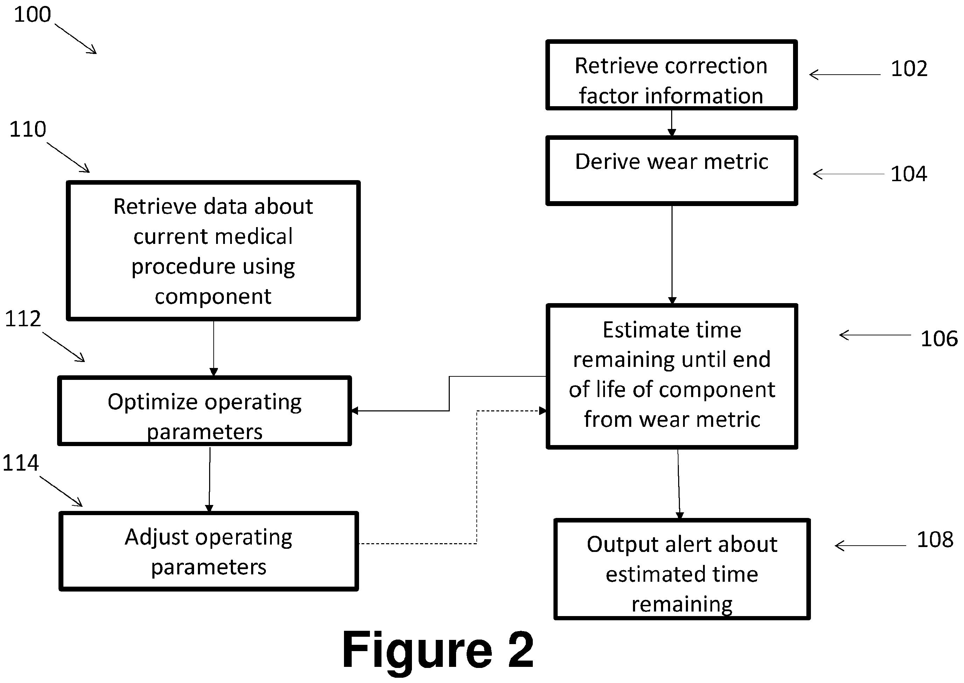

With reference to FIGURE 2, an illustrative embodiment of an instance of the monitoring method **100** is diagrammatically shown as a flowchart. At an operation **102,** information about a correction factor **40** applied during operation of the X-ray tube **10** is retrieved (i.e., from the non-transitory computer readable medium **26**). In some embodiments, the correction factor **40** comprises a filament current correction factor for correcting a filament current $I_f$ of the X-ray tube **10.** The retrieved information about the filament current correction factor **40** can include, for example, X-ray tube calibration data generated by manual calibration of the X-ray tube **10.**

At an operation **104,** a wear metric indicative of wear of a portion of the X-ray tube **10** is derived from the retrieved information about the correction factor **40.** For example, the wear metric is derived from the X-ray tube calibration data. In some embodiments, the wear metric comprises a time derivative of the filament current correction factor **40.**

At an operation **106,** a time remaining until end-of-life (EOL) of the X-ray tube **10** is estimated. The estimation of the time remaining until EOL of the X-ray tube **10** is performed in response to the wear metric satisfying a predetermined adjustment criterion (which can be stored in the non-transitory computer readable medium **26**). The estimating of the time until EOL can also include determining whether wear data or time data is missing from the retrieved information.

At an operation **108,** an alert **30** indicating estimated time remaining until EOL of the X-ray tube **10** is output (i.e., on the display device **24** of the electronic processing device **18**). The alert **30** can be output in response to the estimated time remaining until EOL being less than a predetermined tube replacement alerting threshold time (which can be stored in the non-transitory computer readable medium **26**). In some embodiments, the alert **30** can also include any data that is determined to be missing.

In some embodiments, the electronic processing device **18** can receive alerts **30** from a medical device fleet that includes the medical device **1.** In such embodiments, a representation of all of the alerts **30** can be displayed on the GUI **28** of the display device **24.**

In some embodiments, the method **100** can account for information about a current medical procedure being performed using the medical imaging device **1** before the time remaining until EOL estimation operation **106.** At an operation **110,** information about a current medical procedure regarding the correction factor **40** is retrieved (i.e., from the non-transitory computer readable medium **26**). At an operation **112,** one or more operating parameters of the X-ray tube **10** are optimized from the retrieved information about the component and the retrieved information about the current medical procedure. The operating parameters can include, for example, one or more of the tube voltage **Vt;** the filament current $I_f$; an exposure time for the patient during the current medical procedure, a focal spot size during the current medical procedure, and so forth. The optimization may be based at least in part, for example, on the estimated time until EOL generated at operation **106.** For example, in the case of an X-ray tube, as the estimated time until EOL of the X-ray tube approaches measures can be taken such as reducing the operating parameters (e.g., tube current, exposure time or their product, kV, focal spot and collimation within a certain boundary conditions of the available protocols) in order to extend the life of the X-ray tube. Furthermore, one can also match the image formation algorithms to match the potential image quality degradation that is induced by focusing on the EOL. For example, if the x-ray tube current is reduced bringing in more noise into the resulting image, an additional image de-noising step can be performed tailored to this particular form of degradation. These parameters can be stored in a configuration table **42** stored in the non-transitory computer readable medium **26.** At an operation **114,** the parameter(s) are adjusted based on the optimization operation **112.** To do so, the parameters stored in the configuration table **42** are updated with the optimized parameters. Optionally, the adjusted operating parameters may be fed back to the operation **106** so that the tube life-extending measures can be considered in estimating the time until EOL of the X-ray tube. In some embodiments, while the method **100** is described in terms of a single instance (i.e., a one-time monitoring of the X-ray tube **10),** the wear metric can be derived from a batch of monitoring cases of the component of the medical device **1,** and used to estimate the EOL of the component of the medical device **1.**

EXAMPLE

Some illustrative embodiments of the deriving of the wear metric are described in more detail. A complete usage history of a set of failed filaments is used explicitly with the correction factor or c-factor. By fitting a range of weighted functions that sum the contributions of all individual runs of a given filament, a function is selected for which the average accumulated wear of all failed filaments equals 1.0 as well as possible, and, more importantly, for which the corresponding standard deviation is close to 0.0. As parameters, the applied tube voltage, tube current, c-factor, and run duration can be used to fit the function.

By using such a wear model, the wear of a filament can be monitored, and whenever its accumulated wear approaches 1.0, one can proactively replace it, before it actually breaks. This avoids unplanned downtime. Alternatively, it can be used to proactively ship a new X-ray tube to a system for which the filament is likely to fail in the near future, so that the tube can be replaced shortly after failure. Additionally, the wear model can be used to decrease stock inventory costs, as one can better forecast the number of required new tubes for the near future.

The wear model disclosed herein aims to adapt the contribution of a single run to the accumulated wear by multiplying it with a multiplication factor (i.e., the c-factor) given by $c^{-\alpha}$, for a suitably chosen $\alpha > 1$. As $c < 1$, the multiplication factor $c^{-\alpha}$ is larger than 1. Hence, the wear can increase at a higher rate near the end of life. Alternatively, derivative of the c-factor can be used to obtain a better wear model, by using a multiplication factor given by:

$$\left(-\frac{dc}{dt}\right)^{-\alpha}$$

or

$$\left(-\frac{dc}{dw}\right)^{-\alpha}$$

for a suitably chosen $\alpha$. In the first expression the derivate is taken with respect to time $t$, and in the second expression the derivate is taken with respect to the wear w, for a suitably chosen wear.

Let $f$ denote a filament of an X-ray tube **10,** and let a sequence $R(f)$ of successive runs executed with a filament $f$ be denoted by $R(f) = (r_1(f), r_2(f), ... , r_{n_f}(f))$. Each run $r \in R(f)$ has a run length $l(r)$ and a setting $s(r)$. Setting $s(r)$ specifies tube voltage $V_t(r)$ and tube current $I_t(r)$ applied during run $r$.

A first model (i.e., model $M_1$) is a basic model that does not consider the c-factor. Given a set $F$ of failed filaments for which we have the complete history of runs, model $M_1$ can be constructed as follows. Let $S(F)$ denote the set of settings that have been applied in any of the runs of the filaments in $F$. A number of settings (i.e., the number of different combinations of tube voltage and tube current) is relatively small. If $|S(F)|$ is relatively large, then the settings can be additionally grouped into subsets of similar settings. For simplicity, the number of settings occurring in $F$ can be assumed to be relatively small.

For each setting $s \in S(F)$ a weight $w(s)$ is introduced, and the accumulated wear at failure $w_{af,1}(f)$ of a filament $f \in F$ is defined according to Equation 1:

$$w_{\text{af},1}(f) = \sum_{r \in R(f)} w\big(s(r)\big) \cdot l(r) \qquad (1)$$

The weights w(s) for the settings in $S(F)$ are now chosen such that $w_{af,1}(f)$ are as close to 1.0 as possible for all filaments $f$ in $F$. This fitting will generally not be that well, as the information that is captured in the c-factor is not considered.

A second model (i.e., model $M_2$) additionally considers the c-factor. For this the c-factor $c(r)$ is available for each run $r \in R(f)$ for all filaments $f \in F$. A straightforward adaptation of the first model gives the second model as Equation 2:

$$w_{\text{af},2}(f) = \sum_{r \in R(f)} c(r)^{-\alpha} \cdot w\big(s(r)\big) \cdot l(r) \qquad (2)$$

Now, the weights $w(s)$ and coefficient $\alpha$ are chosen such that $w_{af,2}(f)$ are as close to 1.0 as possible for all filaments $f$ in $F$.

A third model (i.e., model $M_3$) considers the derivate of the c-factor $c(r)$ as this is assumed to be a better indication of the speed that the filament is wearing of during run $r_i(f)$. For ease of notation, the runs $r_{i-k}, ..., r_{i+k}$ are from filament $f$ are not explicitly indicated. To compute the negative derivative of the c-factor, $\Delta_k c(r_i)$ is introduced in Equation 3:

$$\Delta_k c(r) = \frac{c(r_{i-k}) - c(r_{i+k})}{w_2(r_{i-k}, ..., r_{i+k})} \qquad (3)$$

where $w_2(r_{i-k}, ..., r_{i+k})$ is wear accumulated over runs $r_{i-k}, ..., r_{i+k}$ using wear model $M_2$ and where $k$ is chosen to obtain a sufficiently stable derivate of the c-factor with respect to the wear.

Using model $M_3$, the accumulated wear of a filament $f$ is now given by Equation 4:

$$w_{\mathrm{af},3}(f) = \sum_{r \in R(f)} \Delta_k c(r)^{-\alpha} \cdot w\big(s(r)\big) \cdot l(r) \tag{4}$$

where the values of $\alpha$ and multiple weights w are chosen to best fit the data. The accumulated wear at failure of all filaments $f \in F$ should be chosen such that the average difference between the accumulated wear and 1.0 should be zero, and additionally, the corresponding standard deviation should be as close to zero as possible.

Over time, the set of failed filaments for which the complete usage history is available can be extended, and the wear model can be re-fitted, to obtain better and better wear models. Hence, it is important to collect detailed usage data of filaments and store it on a server, to easily recompute the wear model.

The accumulated wear is computed centrally, based on usage data that are repeatedly sent in batches from the medical device to a central location. The used wear model can be updated regularly when the complete usage history of more failed filaments has become available. There can be some noise in the data of individual filaments, so using a large training set of failed filaments to fit the wear model is likely to improve the model's performance. For that reason, it makes sense to compute the accumulated wear centrally, as use can be made of the most recent model.

It is beneficial to have the complete usage history of a failed filament to be able to improve the wear model over time. However, occasionally there can be gaps in the usage data that arrive at the central location. There are two main reasons for possible gaps in the data: (1) the medical device is not used for a given interval of time (e.g., a holiday week); or (2) the medical device was used but the related usage data did not arrive at the central location due to communication or storage issues. The idea is to also compute the accumulated wear locally at the medical device n. This local calculation of the accumulated wear can use an older (probably less accurate) wear model than the wear model that can be used when the complete usage history of the filament is available, but it will be a fair approximation of the contribution to the accumulated wear.

To further optimize the length of time until EOL of the X-ray tube 10, data is collected during tube manufacturing, e.g., tests executed during tube development, tube lifetime tests, etc., which are executed under controlled conditions. Additionally, the data can be complemented from remote serviceable equipment, which is connected with a data retrieval and storage infrastructure that gathers logfiles and other information from the imaging equipment using x-ray tubes and stores them for later analysis. Technical information extracted from the logfiles, like tube type, tube serial number, number of exposures, type of protocols, duration of exposures, focal spot used, tube voltage and currents (requested and applied) but also parameters like patient age and patient weight are used for the statistical analysis with the goal to correlate the control parameters to tube lifetime. Furthermore, information about tube exchanges is required, e.g., from tube serial number changes, and additionally service work orders (SWOs) in case the tube serial number is only manually maintained by the service personnel.

The analysis needs to be repeated for different imaging modalities as well as if tubes type (e.g., MRC CT, CTR17xx, or CTR21xx) and tube usage (i.e., radiography vs. fluoroscopy mode) differs very much, so that the various tube control parameters can be determined for the various different configurations. Alternatively, the problem can also be analyzed integrally in case all relevant above-mentioned parameters are used during the optimization procedure, as clustering based algorithms support the differentiation of the data for the various conditions.

The non-transitory computer readable medium 26 stores the various tube types, tube usages, equipment types, and service contracts etc. together with tube control parameters. Additionally, relevant information about patients is stored like age, size, height, etc.

Either conventional SW algorithms or neural networks (NNs), by training a CNN/RNN based AI algorithms, are used to select the optimal tube parameters for a given input vector consisting of e.g., the before mentioned parameters. The output vector contains the tube control parameters as e.g., the tube voltage, tube current, duration of exposures, and focal spot size. As the tube wear is a strongly non-linear process a slightly lower acquisition time and the use of lower tube currents used for the exposure prolong the tube's lifetime. Another important parameter is the focal spot size used. For the specific patent the larger spot size could be used as it is limited by focal spot size, while the smaller one wears the tube more.

If camera information is available this would enable even better optimization as well as protocol selection as it would enable to complement the logfile information or would allow to verify correctness of the information from the logfile information of e.g., manually entered information by the equipment operator.

Additionally, the information is transmitted to the central data storage thus informing remote service about tube degradation so that steps for the tube replacement can be initiated.

The GUI **28** would inform the medical staff about the state of the tube, including potential degradation, which could impose certain restrictions on the system usage in the near future. This may include restricting voltage, current, exposure time ranges as well as focal spot size. This can manifest in a so called "protocol recommendation", e.g., for extremities a given protocol is preferable in terms of tube life instead of the one currently being used.

Additionally, the customer is informed when certain parameters of lifetime contract are approached or violated.

A focal spot is the area of the anode surface which receives the beam of electrons from the cathode. It is the apparent source of x-rays. In current designs, a small focus filament wears off faster than the large focus one. Today a large number of x-ray tubes have two filaments, e.g., one backup filament for high reliability systems but in most cases to support a small as well as a large focal spot. A size and shape of the focal spot is determined by the size and shape of the electron beam when it strikes the anode. A size shape of the electron beam is determined by dimensions of the filament tungsten coil, construction of a focusing cup, a position of the filament in the focusing cup, and an electric field created between the cathode and anode (i.e., the focal spot enlarges as current increases due to the repulsion of adjacent electrons (i.e., a blooming effect)).

To produce sharp images, focal spots need to be small but able to withstand heat loading without melting the anode target. A small focal spot is used when spatial resolution is important, while a large focal spot is employed when a short exposure time is the priority. The focal spot sizes commonly employed are 0.3 mm and 0.6 mm (i.e., for mammography) and 1.0 mm and 1.2 mm (i.e., for general radiography). Consequently, the energy per $mm^2$ deposited on the anode is larger so that target micro-cracking is increased leading to a reduction in the overall tube lifetime. Consequently, an adjustment to extend the X-ray tube life may include using a large focal spot in favor of a small focal spot when the large focal spot is sufficient for the clinical imaging procedure being performed.

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims.

**Claims**

1. A non-transitory computer readable medium **(26)** storing instructions executable by at least one electronic processor **(20)** to perform a method **(100)** of monitoring a component **(10)** of a medical device **(1),** the medical device **(1)** being a medical imaging device, and the component **(10)** being an X-ray tube of the medical imaging device, the method comprising:

   retrieving information about a filament current correction factor **(40)** applied during operation of the component from the medical device, the filament current correction factor **(40)** being a multiplication factor for adjusting a filament current of the X-ray tube in order to compensate for a varying filament resistance so as to retain a desired X-ray tube current;
   deriving a wear metric indicative of wear of a portion of the component from the retrieved information about the correction factor applied during the operation of the component, the wear metric comprising a time derivative of the filament current correction factor **(40);**
   estimating a time remaining until end-of-life (EOL) of the component based on the wear metric; and
   outputting an alert **(30)** indicating estimated time remaining until EOL of the component.

2. The non-transitory computer readable medium **(26)** of claim 1, wherein:
   the alert **(30)** indicating the estimated time remaining until EOL of the component is output in response to the estimated time remaining until EOL being less than a predetermined tube replacement alerting threshold time.

3. The non-transitory computer readable medium **(26)** of claim 2, wherein the medical imaging device **(1)** comprises a computed tomography (CT) scanner, a C-arm scanner, or a fluoroscope imaging device.

4. The non-transitory computer readable medium **(26)** of claim 2, wherein:

   the retrieved information about the filament current correction factor **(40)** includes X-ray tube calibration data generated by manual calibration of the X-ray tube **(10);** and
   the wear metric is derived from the X-ray tube calibration data.

5. The non-transitory computer readable medium **(26)** of claim 1, wherein the wear metric comprises:

$$\left(-\frac{dc}{dt}\right)^{-\alpha}$$

wherein c denotes the filament current correction factor and $\dfrac{dc}{dt}$ represents a derivative of the filament current correction factor over time and $\alpha$ is a constant.

6.  The non-transitory computer readable medium (26) of claim 1, wherein the wear metric comprises:

$$\left(-\frac{dc}{dw}\right)^{-\alpha}$$

wherein c denotes the filament current correction factor and $\dfrac{dc}{dw}$ represents a derivative of the filament current correction factor over time with respect to a filament wear value for a filament of the X-ray tube and $\alpha$ is a constant.

7.  The non-transitory computer readable medium (26) of any one of claims 1, 5, and 6, wherein estimating a time remaining until an end-of-life (EOL) of the X-ray tube (10) includes:

    determining whether wear data or time data is missing from the retrieved information; and
    outputting the alert (30) indicating the determined missing data.

8.  The non-transitory computer readable medium (26) of any one of claims 1 - 7, wherein estimating the time remaining until EOL of the component (10) is performed in response to the wear metric satisfying a predetermined manual adjustment criterion.

9.  The non-transitory computer readable medium (26) of any one of claims 1 - 8, wherein outputting the alert (30) includes:
    outputting the alert on a display device (24) of a remote monitoring workstation (18).

10. The non-transitory computer readable medium (26) of claim 9, wherein the remote monitoring workstation (18) receives alerts from a medical device fleet that includes the medical device (1), and the method (100) further comprises:
    presenting a representation of the alerts from the medical device fleet on a graphical user interface (GUI) (28) provided on the display device (24).

11. The non-transitory computer readable medium (26) of any one of claims 1 - 10, wherein deriving a wear metric indicative of wear of a portion of the component (10) from the retrieved information about the correction factor (40) applied during the operation of the component includes:
    deriving the wear metric from a batch of cases of monitoring the component.

12. The non-transitory computer readable medium (26) of any one of claims 1-11, wherein the method (100) further comprises:

    retrieving information about a medical procedure to be performed with the medical device and using the component;
    selecting values for one or more operating parameters of the component of the medical device based on the estimate of the time remaining until EOL of the component and the retrieved information about the current medical procedure; and
    adjusting the one or more parameters of the component of the medical device to the selected values.

13. The non-transitory computer readable medium (26) of claim 12, wherein the adjusting includes:
    setting entries for the one or more operating parameters in a configuration table (42) of the medical imaging device (1) to the selected values.

14. The non-transitory computer readable medium (26) of either one of claims 12 and 13, wherein the operating parameters include one or more of: a tube voltage of the X-ray tube (10); a current of a filament (15) of the X-ray tube; an exposure time for the patient during the current medical procedure, and a focal spot selection during the current medical procedure.

**15.** The non-transitory computer readable medium **(26)** of any one of claims 12 - 14, wherein the medical imaging device **(1)** comprises a computed tomography (CT) scanner, a C-arm scanner, or a fluoroscope imaging device.

**Patentansprüche**

**1.** Nichtflüchtiges computerlesbares Medium **(26),** das Anweisungen speichert, die von mindestens einem elektronischen Prozessor **(20)** ausführbar sind, um ein Verfahren **(100)** zur Überwachung einer Komponente **(10)** einer medizinischen Vorrichtung **(1)** durchzuführen, wobei die medizinische Vorrichtung **(1)** eine medizinische Bildgebungsvorrichtung ist und die Komponente **(10)** eine Röntgenröhre der medizinischen Bildgebungsvorrichtung ist, wobei das Verfahren Folgendes umfasst:

Abrufen von Informationen über einen Heizstromkorrekturfaktor **(40),** der während des Betriebs der Komponente aus der medizinischen Vorrichtung angewendet wird, wobei der Heizstromkorrekturfaktor **(40)** ein Multiplikationsfaktor zum Anpassen eines Heizstroms der Röntgenröhre ist, um einen variierenden Heizwiderstand so auszugleichen, dass ein gewünschter Röntgenröhrenstrom beibehalten wird;
Ableiten einer Verschleißmetrik, die den Verschleiß eines Abschnitts der Komponente angibt, aus den abgerufenen Informationen über den während des Betriebs der Komponente angewendeten Korrekturfaktor, wobei die Verschleißmetrik eine zeitliche Ableitung des Heizstromkorrekturfaktors **(40)** umfasst;
Schätzen einer verbleibenden Zeit bis zum Ende der Lebensdauer (EOL) der Komponente basierend auf der Verschleißmetrik; und
Ausgeben einer Warnung **(30),** die die geschätzte verbleibende Zeit bis zum EOL der Komponente angibt.

**2.** Nichtflüchtiges computerlesbares Medium **(26)** nach Anspruch 1, wobei:
die Warnung **(30),** die die geschätzte verbleibende Zeit bis zum EOL der Komponente angibt, als Reaktion darauf ausgegeben wird, dass die geschätzte verbleibende Zeit bis zum EOL weniger ist als eine vorbestimmte Warnschwellenzeit für den Röhrenaustausch.

**3.** Nichtflüchtiges computerlesbare Medium **(26)** nach Anspruch 2, wobei die medizinische Bildgebungsvorrichtung **(1)** einen Computertomographie-(CT)-Scanner, einen C-Bogen-Scanner oder eine Fluoroskopie-Bildgebungsvorrichtung umfasst.

**4.** Nichtflüchtiges computerlesbares Medium **(26)** nach Anspruch 2, wobei:

die abgerufenen Informationen über den Heizstromkorrekturfaktor **(40)** Röntgenröhrenkalibrierungsdaten einschließen, die durch manuelle Kalibrierung der Röntgenröhre **(10)** erzeugt wurden; und
die Verschleißmetrik aus den Kalibrierungsdaten der Röntgenröhre abgeleitet wird.

**5.** Nichtflüchtiges computerlesbares Medium **(26)** nach Anspruch 1, wobei die Verschleißmetrik weiter Folgendes umfasst:

$$\left(-\frac{dc}{dt}\right)^{-\alpha}$$

wobei c den Heizstromkorrekturfaktor bezeichnet und $\frac{dc}{dt}$ eine Ableitung des Heizstromkorrekturfaktors über die Zeit darstellt und $\alpha$ eine Konstante ist.

**6.** Nichtflüchtiges computerlesbares Medium **(26)** nach Anspruch 1, wobei die Verschleißmetrik weiter Folgendes umfasst:

$$\left(-\frac{dc}{dw}\right)^{-\alpha}$$

wobei c den Heizstromkorrekturfaktor bezeichnet und $\frac{dc}{dw}$ eine Ableitung des Heizstromkorrekturfaktors über die Zeit in Bezug auf einen Verschleißwert für einen Heizfaden der Röntgenröhre darstellt und $\alpha$ eine Konstante ist.

7. Nichtflüchtiges computerlesbares Medium **(26)** nach einem der Ansprüche 1, 5 und 6, wobei Schätzen einer verbleibenden Zeit bis zum Ende der Lebensdauer (EOL) der Röntgenröhre **(10)** Folgendes einschließt:

   Bestimmen, ob Verschleißdaten oder Zeitdaten aus den abgerufenen Informationen fehlen; und
   Ausgeben der Warnung **(30),** die die bestimmten fehlenden Daten angibt.

8. Nichtflüchtiges computerlesbares Medium **(26)** nach einem der Ansprüche 1-7, wobei Schätzen der verbleibenden Zeit bis zum EOL der Komponente **(10)** als Reaktion darauf durchgeführt wird, dass die Verschleißmetrik ein vorbestimmtes manuelles Anpassungskriterium erfüllt.

9. Nichtflüchtiges computerlesbares Medium **(26)** nach einem der Ansprüche 1-8, wobei Ausgeben der Warnung **(30)** Folgendes einschließt:
   Ausgeben der Warnung auf einer Anzeigevorrichtung **(24)** einer Fernüberwachungs-Workstation **(18).**

10. Nichtflüchtiges computerlesbares Medium **(26)** nach Anspruch 9, wobei die Fernüberwachungs-Workstation **(18)** Warnungen von einer Flotte medizinischer Vorrichtungen empfängt, die die medizinische Vorrichtung **(1)** einschließt, und wobei das Verfahren **(100)** weiter Folgendes umfasst:
    Präsentieren einer Darstellung der Warnungen von der Flotte medizinischer Vorrichtungen auf einer grafischen Benutzeroberfläche (GUI) **(28),** die auf der Anzeigevorrichtung **(24)** bereitgestellt wird.

11. Nichtflüchtiges computerlesbares Medium **(26)** nach einem der Ansprüche 1-10, wobei Ableiten einer Verschleißmetrik, die Verschleiß eines Abschnitts der Komponente **(10)** angibt, aus den abgerufenen Informationen über den Korrekturfaktor **(40),** der während des Betriebs der Komponente angewendet wird, Folgendes einschließt:
    Ableiten der Verschleißmetrik aus einem Satz von Überwachungsfällen der Komponente.

12. Nichtflüchtiges computerlesbares Medium **(26)** nach einem der Ansprüche 1-11, wobei das Verfahren **(100)** weiter Folgendes umfasst:

    Abrufen von Informationen über einen medizinischen Eingriff, der mit der medizinischen Vorrichtung und unter Verwendung der Komponente durchgeführt werden soll;
    Auswählen von Werten für einen oder mehrere Betriebsparameter der Komponente der medizinischen Vorrichtung basierend auf der Schätzung der verbleibenden Zeit bis zum EOL der Komponente und den abgerufenen Informationen über den aktuellen medizinischen Eingriff; und
    Anpassen des einen oder der mehreren Parameter der Komponente der medizinischen Vorrichtung auf die ausgewählten Werte.

13. Nichtflüchtiges computerlesbares Medium **(26)** nach Anspruch 12, wobei das Anpassen weiter Folgendes einschließt:
    Einstellen von Einträgen für den einen oder die mehreren Betriebsparameter in eine Konfigurationstabelle **(42)** der medizinischen Bildgebungsvorrichtung **(1)** auf die ausgewählten Werte.

14. Nichtflüchtiges computerlesbares Medium **(26)** nach einem der Ansprüche 12 und 13, wobei die Betriebsparameter eines oder mehreres einschließen von: einer Röhrenspannung der Röntgenröhre **(10);** einem Strom eines Heizfadens **(15)** der Röntgenröhre; einer Belichtungszeit für den Patienten während des aktuellen medizinischen Eingriffs und einer Brennpunktauswahl während des aktuellen medizinischen Eingriffs.

15. Nichtflüchtiges computerlesbares Medium **(26)** nach einem der Ansprüche 12-14, wobei die medizinische Bildgebungsvorrichtung **(1)** einen Computertomographie-(CT)-Scanner, einen C-Bogen-Scanner oder eine Fluoroskopie-Bildgebungsvorrichtung umfasst.

**Revendications**

1. Support non transitoire lisible par ordinateur **(26)** stockant des instructions exécutables par au moins un processeur électronique **(20)** pour mettre en œuvre un procédé **(100)** de surveillance d'un composant **(10)** d'un dispositif médical **(1),** le dispositif médical **(1)** étant un dispositif d'imagerie médicale, et le composant **(10)** étant un tube à rayons X du dispositif d'imagerie médicale, le procédé comprenant :

la récupération d'informations sur un facteur de correction de courant de filament **(40)** appliqué pendant le fonctionnement du composant à partir du dispositif médical, le facteur de correction de courant de filament **(40)** étant un facteur de multiplication pour ajuster un courant de filament du tube à rayons X afin de compenser une résistance de filament variable de manière à conserver un courant de tube à rayons X souhaité ;

la dérivation d'une mesure d'usure indicative de l'usure d'une partie du composant à partir des informations récupérées sur le facteur de correction appliqué pendant le fonctionnement du composant, la mesure d'usure comprenant une dérivée temporelle du facteur de correction du courant de filament **(40)** ;

l'estimation du temps restant jusqu'à la fin de vie **(EOL)** du composant sur la base de la mesure d'usure ; et

l'émission d'une alerte **(30)** indiquant le temps estimé restant jusqu'à l'EOL du composant.

2. Support non transitoire lisible par ordinateur **(26)** selon la revendication 1, dans lequel :

l'alerte **(30)** indiquant le temps estimé restant jusqu'à l'EOL du composant est émise en réponse au fait que le temps estimé restant jusqu'à l'EOL est inférieur à un temps seuil d'alerte de remplacement de tube prédéterminé.

3. Support non transitoire lisible par ordinateur **(26)** selon la revendication 2, dans lequel le dispositif d'imagerie médicale **(1)** comprend un scanner de tomodensitométrie **(CT),** un scanner à arceau en C ou un dispositif d'imagerie à fluoroscope.

4. Support non transitoire lisible par ordinateur **(26)** selon la revendication 2, dans lequel :

les informations récupérées sur le facteur de correction de courant de filament **(40)** incluent des données d'étalonnage de tube à rayons X générées par l'étalonnage manuel du tube à rayons X **(10)** ; et

la mesure d'usure est dérivée des données d'étalonnage de tube à rayons X.

5. Support non transitoire lisible par ordinateur **(26)** selon la revendication **1,** dans lequel la mesure d'usure comprend :

$$\left(-\frac{dc}{dt}\right)^{-\alpha}$$

dans lequel c désigne le facteur de correction de courant de filament et $\frac{dc}{dt}$ représente une dérivée du facteur de correction de courant de filament au fil du temps et $\alpha$ est une constante.

6. Support non transitoire lisible par ordinateur **(26)** selon la revendication 1, dans lequel la mesure d'usure comprend :

$$\left(-\frac{dc}{dw}\right)^{-\alpha}$$

dans lequel c désigne le facteur de correction de courant de filament et $\frac{dc}{dw}$ représente une dérivée du facteur de correction de courant de filament au fil du temps par rapport à une valeur d'usure de filament pour un filament du tube à rayons X et $\alpha$ est une constante.

7. Support non transitoire lisible par ordinateur **(26)** selon l'une quelconque des revendications 1, 5 et 6, dans lequel l'estimation du temps restant jusqu'à la fin de vie (EOL) du tube à rayons X **(10)** inclut :

la détermination du fait que les données d'usure ou les données de temps manquent ou non dans les informations récupérées ; et

l'émission de l'alerte **(30)** indiquant les données manquantes déterminées.

8. Support non transitoire lisible par ordinateur **(26)** selon l'une quelconque des revendications 1-7, dans lequel l'estimation du temps restant jusqu'à l'EOL du composant **(10)** est effectuée en réponse au fait que la mesure d'usure satisfait à un critère d'ajustement manuel prédéterminé.

9. Support non transitoire lisible par ordinateur **(26)** selon l'une quelconque des revendications 1-8, dans lequel l'émission de l'alerte **(30)** inclut :
l'émission de l'alerte sur un dispositif d'affichage **(24)** d'un poste de travail de surveillance à distance **(18).**

10. Support non transitoire lisible par ordinateur **(26)** selon la revendication 9, dans lequel le poste de travail de surveillance à distance **(18)** reçoit des alertes d'un parc de dispositifs médicaux qui inclut le dispositif médical **(1),** et le procédé **(100)** comprend en outre :
la présentation d'une représentation des alertes du parc de dispositifs médicaux sur une interface utilisateur graphique (GUI) **(28)** prévue sur le dispositif d'affichage **(24).**

11. Support non transitoire lisible par ordinateur **(26)** selon l'une quelconque des revendications 1-10, dans lequel la dérivation d'une mesure d'usure indicative de l'usure d'une partie du composant **(10)** à partir des informations récupérées sur le facteur de correction **(40)** appliqué pendant le fonctionnement du composant inclut :
la dérivation de la mesure d'usure d'un lot de cas de surveillance du composant.

12. Support non transitoire lisible par ordinateur **(26)** selon l'une quelconque des revendications 1-11, dans lequel le procédé **(100)** comprend en outre :

la récupération d'informations sur une procédure médicale à effectuer avec le dispositif médical et l'utilisation du composant ;
la sélection de valeurs pour un ou plusieurs paramètres de fonctionnement du composant du dispositif médical sur la base de l'estimation du temps restant jusqu'à l'EOL du composant et des informations récupérées sur la procédure médicale en cours ; et
l'ajustement des un ou plusieurs paramètres du composant du dispositif médical aux valeurs sélectionnées.

13. Support non transitoire lisible par ordinateur **(26)** selon la revendication 12, dans lequel l'ajustement inclut :
la définition d'entrées pour les un ou plusieurs paramètres de fonctionnement dans une table de configuration **(42)** du dispositif d'imagerie médicale **(1)** aux valeurs sélectionnées.

14. Support non transitoire lisible par ordinateur **(26)** selon l'une quelconque des revendications 12 et 13, dans lequel les paramètres de fonctionnement incluent un ou plusieurs des éléments suivants : une tension de tube du tube à rayons X **(10)** ; un courant d'un filament **(15)** du tube à rayons X ; une durée d'exposition pour le patient pendant la procédure médicale en cours, et une sélection de point focal pendant la procédure médicale en cours.

15. Support non transitoire lisible par ordinateur **(26)** selon l'une quelconque des revendications 12-14, dans lequel le dispositif d'imagerie médicale **(1)** comprend un scanner de tomodensitométrie (CT), un scanner à arceau en C ou un dispositif d'imagerie à fluoroscope.

**Figure 1**

EP 4 429 553 B1

**Figure 2**

EP 4 429 553 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2019317144 A1

- WO 2020193103 A1